# EUROPÄISCHE PATENTSCHRIFT

(11) **EP 3 789 181 B1**
(45) Veröffentlichungstag und Bekanntmachung des Hinweises auf die Patenterteilung: **04.09.2024**
(21) Anmeldenummer: 20194737.1
(22) Anmeldetag: 04.09.2020
(51) Int. Cl.: B29C 49/80, B29C 49/46, B29C 49/78, G01M 3/28, B29C 49/36, B29C 49/12, B29C 49/42, B29C 49/02, B29C 49/06, G01M 3/26, A61L 9/16

(54) **VORRICHTUNG UND VERFAHREN ZUM UMFORMEN VON KUNSTSTOFFVORFORMLINGEN ZU KUNSTSTOFFBEHÄLTNISSEN MITTELS STATIONÄRER DRUCKLUFTZUFÜHRUNG**
DEVICE AND METHOD FOR REFORMING PLASTIC PRE-FORMS INTO PLASTIC CONTAINERS BY MEANS OF STATIONARY COMPRESSED AIR SUPPLY
DISPOSITIF ET PROCÉDÉ DE FORMAGE DES PRÉFORMES EN MATIÈRE PLASTIQUE POUR RÉCIPIENTS EN MATIÈRE PLASTIQUE AU MOYEN D'UNE ALIMENTATION EN AIR COMPRIMÉ FIXE

(30) Priorität: 05.09.2019 DE 102019123796
(43) Veröffentlichungstag der Anmeldung: 10.03.2021
(73) Patentinhaber: Krones AG, 93073 Neutraubling (DE)
(72) Erfinder: Rauschendorfer, Thomas, 93073 Neutraubling (DE); Trauner, Christian, 93073 Neutraubling (DE); Olenberg, Philipp, 93073 Neutraubling (DE); Philipp, Thomas, 93073 Neutraubling (DE)
(74) Vertreter: Hannke Bittner & Partner mbB Regensburg

(56) Entgegenhaltungen:
- EP-B1- 0 210 344
- EP-B1- 2 402 143
- EP-B1- 2 692 506
- CN-A- 105 415 649
- CN-A- 110 039 747
- CN-U- 207 717 304
- DE-A1- 102017 215 461
- DE-U1- 202015 106 917
- US-B2- 6 935 163

## Beschreibung

Die vorliegende Erfindung bezieht sich auf eine Vorrichtung und ein Verfahren zur Umformung von Kunststoffvorformlingen zu Kunststoffbehältnissen. Derartige Vorrichtungen und Verfahren sind aus dem Stand der Technik seit Langem bekannt. Hierbei werden erwärmte Kunststoffvorformlinge mittels Druckluft zu Kunststoffbehältnissen expandiert. Üblicherweise erfolgt dieser Umformungsvorgang während einer Bewegung der Kunststoffvorformlinge, beispielsweise während eines Transports mittels eines sogenannten Blasrads.

Die Druckluft wird üblicherweise durch eine stationäre Einrichtung zu Verfügung gestellt. Dabei müssen derartige Drucklufteinheiten unterschiedlichsten Anforderungen genügen. So kann einerseits ein unterschiedlicher Reinheitsgrad bei der Herstellung der Kunststoffbehältnisse erforderlich sein und so können unterschiedliche Filtereinrichtungen erforderlich sein, um die Anlage zu betreiben. Auch ist es möglich, dass in Abhängigkeit von herzustellenden Behältnissen unterschiedliche Druckniveaus Einsatz finden. Auch ist es möglich, dass beispielsweise in Abhängigkeit von einer Größe der zu betreibenden Anlage unterschiedliche Druckluftmengen zur Verfügung gestellt werden müssen.

Eine weitere Problematik tritt auf, wenn es in der jeweiligen Vorrichtung zu Fehlern, beispielsweise zu Leckagen, kommt. Im Stand der Technik ist keine Möglichkeit vorgesehen, in einfacher Weise derartige Leckagen ausfindig zu machen.

Aktuell besteht an einer Umformungseinrichtung, etwa einer Streckblasmaschine keine Möglichkeit, gezielt eine Leckage im Hochdruck-Luft-Bereich zu detektieren. Es können beispielsweise Leckagen von der Einspeisung der Druckluft (insbesondere einem Eingang der Blasmaschine) bis hin zur Blasdüse entstehen, welche nicht hinsichtlich ihres Auftrittsbereich differenziert werden können. Die einzige Möglichkeit die Ursache herauszufinden, besteht derzeit darin, den gesamten Druckluftbereich mit einem definierten Druck zu beaufschlagen, die Schutztür der Maschine zu öffnen und rein nach Gehör und Leckagen mittels Leckage-Spray ausfindig zu machen.

Die Erkennung von Lecks in Blasformmaschinen wird in DE102017215461 A1, DE202015106917U1, CN110039747A und CN105415649B offenbart.

Allgemeine Lecktestgeräte sind in CN207717304U, US6935163B2 und EP0210344B1 offenbart.

Im internen Stand der Technik der Anmelderin wurde bereits an Konzepten für eine automatisierte Leckagedetektion von Domdruckregelventilen, Ringkanälen und Luftdrehverteilern gearbeitet. Eine Auswertung auf Undichtigkeiten der einzelnen Ventile des Ventilblocks oder der Blasdüsen-Lippe oder der Blaskolben-/ Reckstangendichtung wurden jedoch nicht realisiert.

Weder bei der Werksmontage der Umformungsvorrichtung noch während der Außenmontage beim Kunden vor Ort kann mit wenig Aufwand die gesamte Dichtigkeit des Druckluftbereichs geprüft werden. Dennoch kommt es bei den Umformungsvorrichtungen, beispielsweise Streckblasmaschinen, häufig zu Undichtigkeiten, welche aufwändig identifiziert und behoben werden müssen, da diese an diversen Stellen auftreten können.

Außerdem kann der Transport der Maschine verursachen, dass an unterschiedlichsten Stellen Beschädigungen oder Undichtigkeiten auftreten. Dann muss beim Kunden vor Ort erneut nach einer oder mehreren Leckagen gesucht werden. Dies ist ineffizient und kostet besonders in der Außenmontage beim Kunden Zeit und Geld, da bei der Kundenabnahme die spezifizierten Energie- und Luftverbräuche eingehalten werden müssen.

Außerdem kann Stand heute für eine bessere Planung von Überholungen/Instandhaltungen nicht eindeutig auf den Verschleißzustand von Ventilen, Dichtungen etc. geschlossen werden.

Eine weitere Aufgabe der vorliegenden Erfindung besteht daher darin, eine vereinfachte Möglichkeit zu schaffen, um in derartigen Vorrichtungen Leckage festzustellen bzw. aufzuspüren.

Diese Aufgaben werden erfindungsgemäß durch die Gegenstände der unabhängigen Patentansprüche erreicht. Vorteilhafte Ausführungsformen und Weiterbildungen sind Gegenstand der Unteransprüche.

Die Druckluftversorgungseinrichtung kann als eine modular aufgebaute Einrichtung aufgebaut werden, welche eine Be- und/ oder Entlüftungseinrichtung für Hochdruckluft aufweist, wobei diese modular aufgebaute Einrichtung je nach Anwendung mit weiteren Versorgungskomponenten bestückbar ist.

Bei dieser Ausgestaltung wird daher ein modularer Aufbau der Druckluftvesorgungseinrichtung vorgeschlagen. Im Gegensatz zum Stand der Technik, in dem diese Versorgungseinrichtungen jeweils einheitlich aufgebaut sind und nicht nach Anwendungsfall modifiziert werden können, erlaubt die hier beschriebene modular aufgebaute Einheit eine Versorgung der jeweiligen Vorrichtung bzw. auch der Beaufschlagungseinrichtung je nach dem Anwendungsfall.

Die vorliegende Offenbarung ist weiterhin auf eine Vorrichtung zum Umformen von Kunststoffvorformlingen zu Kunststoffbehältnissen gerichtet, die wenigstens eine Umformungsstation aufweist, welche eine Blasform aufweist, innerhalb derer die Kunststoffvorformlinge durch Beaufschlagung mit einem fließfähigen und insbesondere gasförmigen Medium zu den Kunststoffbehältnissen umformbar sind und die weiterhin eine Beaufschlagungseinrichtung aufweist, welche die Kunststoffvorformlinge mit dem fließfähigen Medium beaufschlagt, wobei die Vorrichtung eine Druckluftversorgungseinrichtung aufweist, welche das fließfähige Medium zur Verfügung stellt und diese Druckluftversorgungseinrichtung stationär angeordnet ist.

Bei dieser Ausgestaltung weist die Druckluftversorgungseinrichtung eine Leckageerkennungseinrichtung auf, welche dazu geeignet und bestimmt ist, Leckagen in der Druckluftversorgung der Vorrichtung zu ermitteln.

Es wird jedoch darauf hingewiesen, dass die hier beschriebenen Ausgestaltungen auch miteinander kombiniert sein können.

Bei einer weiteren vorteilhaften Ausführungsform weist die Vorrichtung einen bewegbaren und insbesondere drehbaren Träger auf, an dem die wenigstens eine Umformungsstation angeordnet ist. Bei einer weiteren bevorzugten Ausführungsform weist die Vorrichtung eine Vielzahl von Umformungsstationen auf, die insbesondere an dem besagten drehbaren Träger angeordnet sind. Besonders bevorzugt transportiert der Träger die Umformungsstationen entlang eines kreisförmigen Pfades.

Bei einer weiteren bevorzugten Ausführungsform handelt es sich bei der Beaufschlagungseinrichtung um eine Blasdüse bzw. die Beaufschlagungseinrichtung weist eine Blasdüse auf, welche an eine Mündung der Kunststoffvorformlinge ansetzbar ist, um diese so mit dem gasförmigen Medium zu beaufschlagen.

Bevorzugt weist die Blasform mehrere Blasformteile auf, die zum Öffnen und Schließen der Blasform bezüglich einander bewegbar sind, beispielsweise schwenkbar sind. Besonders bevorzugt sind diese Blasformteile jeweils an Blasformträgerteilen angeordnet.

Bei einer weiteren bevorzugten Ausführungsform weist die Umformungsstation eine Dehneinrichtung auf und insbesondere einen stangenartigen Körper, der in die Kunststoffvorformlinge einführbar ist, um diese in ihrer Längsrichtung zu dehnen.

Bei einer weiteren bevorzugten Ausführungsform weist die Vorrichtung wenigstens ein auf dem bewegbaren und insbesondere drehbaren Träger angeordnetes Druckluftreservoir auf. Bevorzugt kann dieses Druckluftreservoir von der oben erwähnten Druckluftversorgungseinrichtung versorgt werden. Bei einer bevorzugten Ausführungsform ist dieses Druckluftreservoir als Kanal und insbesondere als Ringkanal ausgebildet. Besonders bevorzugt ist dieses Druckluftreservoir dazu geeignet und bestimmt, mehrere Umformungsstationen mit dem gasförmigen Medium zu versorgen.

Bei einer bevorzugten Ausführungsform weist die Vorrichtung wenigstens eine Verteileinrichtung auf, welche die von der Druckluftversorgungseinrichtung stammende Luft auf die einzelnen Umformungsstationen verteilt. Bei dieser Verteileinrichtung kann es sich besonders bevorzugt um einen sogenannten Drehverteiler handeln, der dazu geeignet und bestimmt ist, von einer stationären Quelle, hier der Druckluftversorgungseinrichtung die Druckluft auf die einzelnen Umformungsstationen zu verteilen.

Bei einer bevorzugten Ausführungsform weist die Vorrichtung wenigstens zwei Zuführleitungen auf, welche die Druckluft dem besagten Drehverteiler und insbesondere den besagten Reservoirs zur Verfügung stellt.

Dabei kann es sich bei einer der Druckluftleitungen bzw. Zuführleitungen um eine Hochdruckluftleitung handeln und bei der anderen um eine Niederdruckleitung.

Unter einem hohen Druck wird im Rahmen der vorliegenden Erfindung ein Druck verstanden, der größer ist als 20 bar, bevorzugt größer als 30 bar und besonders bevorzugt größer als 35 bar. Besonders bevorzugt handelt es sich um einen Druck, der kleiner ist als 100 bar, bevorzugt kleiner als 80 bar, bevorzugt kleiner als 60 bar.

Unter einem Niedrigdruck wird ein Druck verstanden, der kleiner ist als 20 bar, bevorzugt kleiner als 15 bar, bevorzugt kleiner als 10 bar.

Bei einer weiteren bevorzugten Ausführungsform weist die Druckluftversorgungseinrichtung eine Vielzahl von Ventileinrichtungen zum Versorgen bzw. zum Betreiben der Druckluftversorgungseinrichtung auf. Bei einer weiteren bevorzugten Ausführungsform weist die Druckluftversorgungseinrichtung eine Druckluftzuführung auf, welche beispielsweise eine Verbindung mit einem Kompressor oder einer Hochdruckleitung darstellen kann.

Bei einer weiteren bevorzugten Ausführungsform weist die Druckluftversorgungseinrichtung auch eine Abführleitung auf, welche insbesondere Druckluft abführen kann und welche insbesondere mit der Vorrichtung zum Umformen von Kunststoffvorformlingen zu Kunststoffbehältnissen verbindbar ist.

Bei einer weiteren bevorzugten Ausführungsform sind die oben genannten Versorgungskomponenten aus einer Gruppe von Komponenten ausgewählt, welche Verbrauchsmesseinrichtungen, Filtereinrichtungen, insbesondere Feinfiltereinrichtungen, Aktivkohlefiltereinrichtung, Staubfiltereinrichtungen, Kondensatabscheider, Vorfiltereinrichtungen, Sterilfiltereinrichtungen und dergleichen aufweist.

Auch ist es möglich, dass diese genannten Komponenten in einer gewünschten Weise bzw. in einer bestimmten Reihenfolge zusammengeschaltet werden. So können beispielsweise unterschiedliche Filtereinrichtungen je nach den gewünschten Anforderungen zusammengeschaltet werden.

Zum Zusammenschalten der einzelnen Komponenten können dabei Elemente verwendet werden, die aus einer Gruppe von Elementen ausgewählt sind, welche O-Ringe, Zugstangen, Befestigungsklammern und der gleichen Enthält. Mittels derartiger Elemente kann eine Modularität hergestellt werden. Daneben können jedoch auch Schraubverbindungen, Steckverbindungen, Bajonettverbindungen und dergleichen verwendet werden.

Auch können unterschiedliche Komponenten an einen Bedarf der Maschine angepasst werden, beispielsweise an einen Druckluftbedarf. Dieser kann beispielsweise von der Anzahl der Umformungsstationen abhängen.

So ist es beispielsweise möglich, dass die Druckluftversorgungseinrichtung in einer einfachsten Ausführungsform eine Be- und Entlüftungseinheit der Hochdruckluft aufweist und einen integrierten Feinfilter. In einer weiteren Ausführungsform kann neben der Be- und Entlüftungseinheit der Hochdruckluft auch eine integrierte Vorfiltrationsbaugruppe vorgesehen sein.

Diese Be- und Entlüftungseinheit dient bevorzugt einerseits dazu, die Vorrichtung zum Umformen von Kunststoffbehältnissen mit Druckluft zu versorgen und andererseits bevorzugt auch dazu, Druckluft aufzunehmen.

In einer weiteren bevorzugten Ausführungsform können auch Sterilfilter und insbesondere autoklavierbare Sterilfilter vorgesehen sein. Auch diese können dabei in das modulare System integrierbar sein. Bevorzugt ist wenigstens ein Sterilfilter und sind bevorzugt mehrere und besonders alle Sterilfilter stromabwärts der Be- und Entlüftungseinheit angeordnet. Auf diese Weise wird erreicht, dass die Be- und Entlüftungseinheit selbst nicht steril ausgeführt zu werden braucht.

In einer weiteren Ausführungsform kann die Vorrichtung, wie unten genauer beschrieben, eine Niederdruckbeaufschlagungseinrichtung aufweisen, um Leckagen zu ordnen. Dabei können insbesondere Leckagen in dem Druckluftsystem einer Blasformmaschine bzw. der Vorrichtung zum Umformen der Kunststoffvorformlinge zu Kunststoffbehältnissen erkannt werden.

Bei einer weiteren bevorzugten Ausführungsform weist die Druckluftversorgungseinrichtung eine Dauerdruckluftversorgung für die Blasmaschine auf, um beispielsweise Ventileinrichtungen der Blasformmaschine zu schalten. Bei diesen Ventileinrichtungen kann es sich beispielsweise um pneumatisch betätigte Ventile handeln. Diese können mittels der Niederdruckdauerluftversorgung aktiv geschalten werden. Diese Dauerluftversorgung kann beispielsweise Druckluft mit einem Druck von 10 bar zur Verfügung stellen.

Bei einer weiteren bevorzugten Ausführungsform weist die Drucklufterzeugungseinrichtung eine Niedrigdruckluftversorgungeinrichtung auf, wie beispielsweise eine 10 bar Druckluftversorgungseinrichtung, welche auch aktiv zu- oder abgeschaltet werden kann.

Bei einer weiteren bevorzugten Ausführungsform ist ein Verbrauchsmesser in die Druckluftversorgungseinrichtung integriert. Dieser Verbrauchsmesser kann beispielsweise unmittelbar an dem Eingang der Druckluftversorgungseinrichtung angeordnet sein. Dieser Verbrauchsmesser kann jedoch auch in einem Rücklauf geschalten sein.

Bei einer weiteren bevorzugten Ausführungsform kann die Be- und Entlüftungseinheit der Hochdruckluft auf unterschiedliche maximale Volumenströme angelegt sein, beispielsweise auf einem Volumenstrom von 6200 Nm³/h. Auch ist es dabei möglich, dass Filterelemente und Filterglocken in unterschiedlichen Größen ausgelegt sein können. Diese können dabei Größen zwischen 1 und 3100 Nm³/h, beispielsweise bei einem Druck von 40 bar, aufweisen oder auch eine zweite Größe, die bis zu 6200 Nm³/h ebenfalls bei 40 bar erreicht.

Bei einer besonders bevorzugten Ausführungsform ist die hier beschriebene modulare Druckluftversorgungseinrichtung so aufgebaut, dass in die Be- und/ oder Entlüftungseinheit beispielsweise in eine Hochdruckbelüftungseinheit eine Filtration integriert ist und/ oder die Filtrationseinrichtung direkt an die Be- und/ oder Entlüftungseinheit montiert werden kann.

Dabei ist es möglich, dass die besagte Be- und Entlüftungseinheit der Hochdruckluft auf einen maximalen Volumenstrom ausgelegt ist, beispielsweise einen maximalen Volumenstrom von zwischen 5000 und 7000 Nm³/h und bei einem Druck zwischen 35 und 45 bar.

Je nach dem benötigten Luftvolumen können Filterelemente und Glocken in unterschiedlichen Größen verwendet werden. So kann beispielsweise eine erste Größe vorgesehen sein, die bis zu einem Volumen von 3500, bevorzugt 3100 m³/h bei einem Druck zwischen 35 und 45 bar geeignet ist und eine zweite Größe, die für ein Luftvolumen bis 6200 Nm³/h bevorzugt ebenfalls bei einem Druck zwischen 35 und 45 bar geeignet ist.

Bevorzugt ist jedoch eine spätere Umrüstung auf einen höheren Volumenstrom möglich.

Bei einer weiteren bevorzugten Ausführungsform weist die Vorrichtung wenigstens eine erste Filtereinrichtung und wenigstens eine zweite Filtereinrichtung auf. Dabei ist es möglich, dass diese Filtereinrichtungen hintereinander geschalten sind. Weiterhin können bevorzugt diese Filtereinrichtungen unterschiedliche Filtergrade aufweisen. So kann es sich beispielsweise bei der ersten Filtereinrichtung um eine Grobfiltereinrichtung und bei der zweiten Filtereinrichtung um eine Feinfiltereinrichtung handeln.

Bei einer weiteren vorteilhaften Ausführungsform weist die Vorrichtung eine Niederdruckluftversorgungseinheit auf. Diese kann wie oben erwähnt zum Schalten von Ventilen eingesetzt werden.

Bei einer weiteren vorteilhaften Ausführungsform ist diese Niederdruckluftversorgungseinheit aktivierbar und deaktivierbar. Dabei kann insbesondere diese Niederdruckluftversorgungseinheit unabhängig von der Be- und Entlüftungseinrichtung aktivierbar und deaktivierbar sein.

Bei einer weiteren vorteilhaften Ausführungsform weist die Druckluftversorgungseinrichtung eine Vielzahl von Ventileinrichtungen auf, wobei diese Ventileinrichtungen insbesondere Luftventileinrichtungen sind und besonders bevorzugt aus einer Gruppe von Ventileinrichtungen ausgewählt sind, welche Kolbenschieberventile, Sitzventile, Kugelhahnventile und dergleichen enthält.

Bei einer weiteren vorteilhaften Ausführungsform weist die Vorrichtung eine Überwachungseinrichtung zum Überwachen der Luftventileinrichtungen auf. So können beispielsweise analoge oder digitale Druckschalter Einsatz finden.

Die vorliegende Offenbarung ist weiterhin auf eine Druckluftversorgungseinrichtung gerichtet, die als eine modular aufgebaute Einrichtung aufgebaut ist, welche eine Be- und/ oder Entlüftungseinrichtung für Hochdruckluft aufweist, wobei diese modular aufgebaute Einrichtung je nach Anwendung mit weiteren Versorgungskomponenten bestückbar ist.

Bei einer weiteren bevorzugten Ausführungsform weist die Druckluftversorgungseinrichtung wenigstens einen Niederdruckanschluss auf. Bevorzugt ist dieser Niederdruckanschluss aktivierbar und deaktivierbar und insbesondere von der Be- und Entlüftungseinrichtung unabhängig aktivierbar und deaktivierbar.

Bei einer weiteren bevorzugten Ausführungsform ist die oben erwähnte Niederdruckluftversorgungseinrichtung mit der Druckluftversorgung der Vorrichtung verbindbar. Damit wird insbesondere eine Strömungstechnische Verbindung beschrieben.

Bei einer weiteren bevorzugten Ausführungsform weist die Leckageerkennungseinrichtung eine Druckanschlusseinrichtung auf, wobei diese Druckanschlusseinrichtung einen gegenüber einer Hauptdruckanschlusseinrichtung verkleinerten Querschnitt aufweist. Bevorzugt beträgt dieser verkleinerte Querschnitt weniger als 90 %, bevorzugt weniger als 80 %, bevorzugt weniger als 70 %, bevorzugt weniger als 60 % des Querschnitts der Hauptdruckanschlusseinrichtung.

Bei einer weiteren vorteilhaften Ausführungsform weist die Vorrichtung eine erste Druckluftzuführungseinrichtung und eine zweite Druckluftzuführungseinrichtung auf. Diese können wie oben erwähnt, unterschiedliche Drücke führen.

Vorteilhaft sind die beiden Druckluftzuführungseinrichtungen unabhängig voneinander regelbar. Besonders bevorzugt sind diese Druckluftzuführungseinrichtungen auch getrennt voneinander absperrbar.

Bei einer weiteren bevorzugten Ausführungsform weist die Vorrichtung eine Verschließeinrichtung zum Verschließen der Beaufschlagungseinrichtung auf. Diese Verschließeinrichtung kann beispielsweise an die Beaufschlagungseinrichtung, wie etwa an eine Blasdüse zum Zweck der Leckagekontrolle angelegt werden und insbesondere diese abdichten.

Bei einer weiteren bevorzugten Ausführungsform weist die Verschließeinrichtung einen Schwenkmechanismus auf, der einen Verschließkörper in einen Verfahrweg der Beaufschlagungseinrichtung schwenkt.

Bevorzugt handelt es sich bei dem Verschließkörper um einen Schließteller. Dieser Verschließkörper beeinflusst bevorzugt im Normal- bzw. Arbeitsbetrieb den Blasprozess nicht. Bei einer besonders bevorzugten Ausführungsform weist dieser Schwenkmechanismus eine magnetisch arbeitende Antriebseinrichtung auf.

Mittels einer stationär einfahrbaren Vorrichtung soll ein Verschließkörper, bevorzugt ein Schließteller, welcher im normalen Zustand, den Blasprozess nicht beeinflusst, eingeklappt werden. Die Klappbewegung wird aus hygienischen Gründen vorzugshalber rein über Magnetkraft durchgeführt.

Sobald die Vorrichtung, d.h. der Verschließkörper vollständig eingeklappt ist (direkt unter die Beaufschlagungseinrichtung bzw. Blasdüse), bewegt sich und insbesondere senkt sich die Blasdüse, um einen geschlossenen Raum zu erzeugen. Dieses definierte Volumen wird bevorzugt anschließend mit Druckluft beaufschlagt, um eine potentielle Leckage zu detektieren.

Bevorzugt wird dabei auch unmittelbar auf die konkrete Stationsnummer hingewiesen, bzw. die Umformungsstation, welche den Prozess durchführt identifiziert. Durch eine festgelegte Leckage-Prüfroutine soll die Dichtigkeit der einzelnen Ventile an jedem Ventilblock geprüft werden.

Zudem kann die Leckage damit, je nach Fehlerfall, auf ein ganz konkretes Bauteil bzw. im schlechtesten Fall auf einige wenige Komponenten eingegrenzt werden. Dadurch kann beispielsweise pro Ventilblock gezielt auf jedes Ventil (P1, Pi, Pi+ P2) oder auf eine Undichtigkeit der Blasdüsen-Lippe/ Blaskolben-/ Reckstangendichtungen oder des Exhaust-Ventils geschlossen werden.

Auf diese Weise ist eine schnellere werkseitige Montage möglich, da eine gezielte und schnelle Fehlerbehebung ermöglicht wird. Probleme mit Dichtungen und vor allem Ventilen des Ventilblocks fallen schon während der werkseitigen Montage auf und die entsprechenden Komponenten können ausgetauscht werden. Auch können Lieferanten bereits frühzeitig über etwaige Fehler informiert werden. Auch können bereits vor der eigentlichen Auslieferung an die Kunden sämtliche Leckagen behoben werden.

Die vorliegende Erfindung ist weiterhin auf ein Verfahren zum Betreiben einer Vorrichtung zum Umformen von Kunststoffvorformlingen zu Kunststoffbehältnissen gerichtet, wobei Kunststoffvorformlinge mit wenigstens einer Umformungsstation zu den Kunststoffbehältnissen umgeformt werden, wobei die Umformungsstation eine Blasform aufweist, innerhalb derer die Kunststoffvorformlinge durch Beaufschlagung mit dem fließförmigen bzw. fließfähigen Medium zu den Kunststoffbehältnissen umgeformt werden und wobei die Kunststoffvorformlinge mit einer Beaufschlagungseinrichtung mit dem fließfähigen Medium beaufschlagt werden, wobei mittels einer Druckluftversorgungseinrichtung das fließfähige Medium zur Verfügung gestellt wird und diese Druckluftversorgungseinrichtung stationär angeordnet ist. Weiterhin wird wenigstens zeitweise die Vorrichtung mit der Druckluftversorgungseinrichtung auf Leckagen hin überprüft.

Besonders bevorzugt erfolgt dieses Überprüfen auf Leckagen hin in einem Überprüfungsbetrieb, der sich von einem Produktionsbetrieb der Vorrichtung unterscheidet.

Erfindungsgemäss erfolgt die Überprüfung auf Leckagen mit einem Niederdruck, also insbesondere einem Druck, der unter 15 bar und bevorzugt unter 10 bar liegt.

Auf diese Weise ist eine Lokalisierung der Leckage möglich. Beispielsweise kann auf diese Weise herausgefunden werden, auf welcher Druckstufe eine Leckage auftritt. Nach dem Ermitteln der Druckstufe können weitere Leckageortungsschritte eingeleitet werden.

Bei einem bevorzugten Verfahren werden in insbesondere aufeinanderfolgenden Verfahrensschritten unterschiedliche Bestandteile der Vorrichtung auf Leckagen hin überprüft. Auf diese Weise können nacheinander unterschiedliche Bestandteile der Vorrichtung und/oder Bereiche der Vorrichtung überprüft werden und so kann schneller die Leckage ermittelt werden.

Bei einem bevorzugten Verfahren werden die Prüfungen und der nachfolgenden Reihenfolge vorgenommen:
- Überprüfen einer Luftdrehverteilereinrichtung
- Überprüfen von Regelventilen und insbesondere Domdruckregelventilen;
- Überprüfen von Druckluftspeichereinrichtungen, insbesondere Ringkanälen;
- Überprüfen von Dichtungseinrichtungen.

Bevorzugt erfolgt daher die Prüfungsreihenfolge in der Richtung, in der auch im Arbeitsbetrieb die Blasluft strömt. Es wäre jedoch auch möglich, eine andere Reihenfolge zu wählen.

So ist es möglich, dass in einem Verfahrensschritt Luft in den Speichereinrichtungen wie insbesondere den Ringkanälen "eingesperrt" wird und über einen vorgegebenen Zeitraum die Drücke gemessen werden. Falls keine Veränderung des Druckes festgestellt wird, kann darauf geschlossen werden, dass in den Ringkanälen und/oder den Leitungen, wie etwa den Verschlauchungen und/oder in den Ventilen keine Leckagen auftreten.

In einem weiteren Verfahrensschritt wird bevorzugt die Beaufschlagungseinrichtung bzw. Blasdüse auf Leckagen hin überprüft. Zu diesem Zweck wird bevorzugt die Beaufschlagungseinrichtung abgeschlossen so dass kein gasförmiges Medium aus dieser austritt. Dies wird vorteilhaft mit der oben beschriebenen Verschließeinrichtung durchgeführt. Bevorzugt wird ein Auslassventil (Exhaust) geschlossen und der nunmehr abgeschlossene Raum mit einem bestimmten Druck, z.B. beispielsweise einem Fertigblasdruck und bevorzugt einem Druck von mehr als 10bar, bevorzugt mehr als 20bar beaufschlagt.

Dabei kann dieses Beaufschlagen durch das Öffnen desjenigen Ventils erfolgen, welches auch in einem Arbeitsbetrieb diesen Druck zuführt. Sobald sich der gewünschte Druck eingestellt hat, wird bevorzugt das betreffende Ventil wieder geschlossen und der Druck in dem abgeschlossenen Raum gehalten. Wenn sich auch in diesem Fall über eine vorgegebene Zeit kein Druckverlust einstellt, kann daraus geschlossen werden, dass auch Elemente wie die Blasdüsen - Lippe, die Kolbenführungsdichtung, die Reckstangendichtung und das Auslassventil dicht sind.

Nachfolgend werden einige Beispiele für auftretende Fehler dargestellt. Diese Beispiele sind jedoch nicht abschließend.

In einem ersten Beispiel wird Luft mit einem definierten Druck in den Ringkanälen eingesperrt und der Druck über einen bestimmten Zeitraum hinweg gemessen. Falls beispielsweise ein Druckabfall in einem Ringkanal P2 detektiert wird kann auf eine Leckage in dem Bereich dieses Ringkanals oder dessen Verschlauchung oder den zugehörigen P2 Ventilen aller Ventilblöcke geschlossen werden.

Um den fehlerhaften Bereich genauer einzugrenzen werden die jeweiligen Beaufschlagungseinrichtungen Blasdüsen geprüft. Zu diesem Zweck wird die bzw. werden die Beaufschlagungseinrichtungen geschlossen und das entsprechende Auslassventil wird ebenfalls geschlossen. Anschließend wird der eingesperrte Raum mit Druck beaufschlagt (etwa über das P1 - Ventil). Nachdem ein bestimmter Druck erreicht wurde schließt dieses Ventil wieder. Falls nun an einer bestimmten Umformungsstation ein Druckanstieg detektiert wird, ist dies ein Indiz dafür, dass das Ventil P2 dieser Station undicht ist.

Bevorzugt ist daher jeder der Umformungsstationen ebenfalls wenigstens ein und bevorzugt eine Vielzahl von Druckmesseinrichtungen zugeordnet.

Bei einem weiteren Fehlerfall wird Luft mit einem definierten Druck in den Ringkanälen eingesperrt und der Druck über einen bestimmten Zeitraum hinweg gemessen. Falls beispielsweise ein Druckabfall in einem Ringkanal Pi detektiert wird kann auf eine Leckage in dem Bereich dieses Ringkanals oder dessen Verschlauchung oder der zugehörigen P2 Ventile aller Ventilblöcke geschlossen werden.

Um den fehlerhaften Bereich genauer einzugrenzen werden die jeweiligen Beaufschlagungseinrichtungen bzw. Blasdüsen geprüft. Zu diesem Zweck wird die bzw. werden die Beaufschlagungseinrichtungen geschlossen und das entsprechende Auslassventil wird ebenfalls geschlossen. Anschließend wird der eingesperrte Raum mit Druck beaufschlagt (etwa über das P2 - Ventil). Nachdem ein bestimmter Druck erreicht wurde schließt dieses Ventil wieder.

Falls nun nach einer gewissen Zeit keine Druckveränderung festgestellt wird lässt sich daraus schließen, dass die Beaufschlagungseinrichtungen bzw. deren Dichtungen dicht sind und keine Leckage über die Pi-Ventile verursacht wird. In diesem Fall ist von einer Leckage in dem Pi - Ringkanal oder dessen Verschlauchung auszugehen.

Bei einem weiteren Fehlerfall wird Luft mit einem definierten Druck in den Ringkanälen eingesperrt und der Druck über einen bestimmten Zeitraum hinweg gemessen. Falls keine Veränderung des Druckes festgestellt wird, treten keine Leckagen im Bereich der Ringkanäle und deren Verschlauchungen und auch nicht im Bereich der Ventile an den Ventilblöcken auf.

In einem nächsten Schritt werden auch hier die Beaufschlagungseinrichtungen geprüft. Zu diesem Zweck wird die bzw. werden die Beaufschlagungseinrichtungen geschlossen und das entsprechende Auslassventil wird ebenfalls geschlossen. Anschließend wird der eingesperrte Raum mit Druck beaufschlagt (etwa über das P2 - Ventil). Nachdem ein bestimmter Druck erreicht wurde schließt dieses Ventil wieder. Wenn nun an einer bestimmten Station ein Druckabfall gemessen wird, bedeutet dies, dass die Blasdüsen-Lippe, die Kolbenführungsdichtung, die Reckstangendichtung oder das Auslassventil dieser betreffenden Umformungsstation undicht ist.

Weitere Vorteile und Ausführungsformen ergeben sich aus den beigefügten Figuren:
Darin zeigen:
- Fig. 1: eine schematische Darstellung einer Vorrichtung zum Umformen von Kunststoffvorformlingen zu Kunststoffbehältnissen;
- Fig. 2: eine Darstellung einer Druckversorgungseinrichtung in einer ersten Ausführungsform;
- Fig. 3: eine Darstellung einer Druckversorgungseinrichtung in einer weiteren Ausführungsform;
- Fig. 4: eine Darstellung einer Druckversorgungseinrichtung in einer weiteren erweiterten Ausführungsform;
- Fig. 5: eine Darstellung eines Funktionsschemas einer Druckversorgungseinrichtung; und
- Fig. 6: eine blockdiagrammartige Darstellung einer erfindungsgemäßen Vorrichtung.

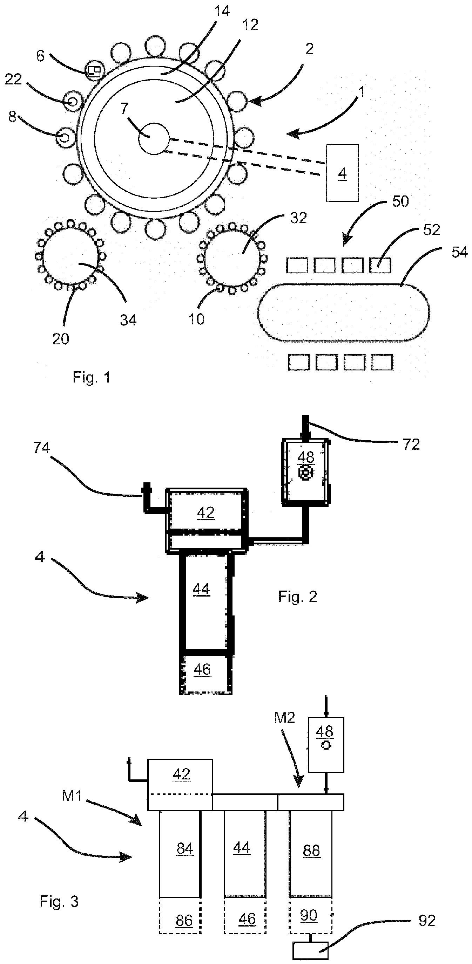

Figur 1 zeigt eine schematische Darstellung einer erfindungsgemäßen Vorrichtung zum Umformen von Kunststoffvorformlingen 10 zu Kunststoffbehältnissen 20. Dabei bezieht sich das Bezugszeichen 50 auf eine Erwärmungseinrichtung, welche die Kunststoffvorformlinge erwärmt. Zu diesem Zweck werden die Kunststoffvorformlinge mit einer Transporteinrichtung 54 transportiert und mit einer Vielzahl von Erwärmungseinrichtungen 52 erwärmt. Diese Erwärmungseinrichtungen sind bevorzugt stationär angeordnet.

Anschließend werden die Kunststoffvorformlinge 10 mittels einer Zuführeinrichtung, wie etwa eines Transportsterns 32 an die eigentliche Umformungseinrichtung 1 übergeben. Diese Umformungseinrichtung 1 weist einen drehbaren Träger 12 auf, an dem eine Vielzahl von Umformungsstationen 2 angeordnet ist. Jede dieser Umformungsstationen weist dabei eine Beaufschlagungseinrichtung 22 auf, wie etwa eine Blasdüse, welche an die Mündungen der Kunststoffvorformlinge anlegbar ist, um diese Druckluft zu beaufschlagen und so zu expandieren.

Weiterhin weist jede Umformungsstation 2 eine Blasform 8 auf, innerhalb derer die Kunststoffvorformlinge zu den Kunststoffbehältnissen expandiert werden.

Weiterhin weisen die Umformungsstationen 2 jeweils Ventileinrichtungen 6 auf, wie etwa Ventilblöcke auf, welche üblicherweise mehrere Ventile aufweisen, um die Kunststoffvorformlinge mit mehreren Druckstufen zu beaufschlagen, wie etwa einem Vorblasdruck, einem Zwischenblasdruck und dergleichen.

Das Bezugszeichen 14 bezieht sich auf ein Druckluftreservoir wie insbesondere aber nicht ausschließlich einen Ringkanal, der die einzelnen Umformungsstationen 2 mit Druckluft versorgen kann.

Das Bezugszeichen 4 kennzeichnet in seiner Gesamtheit eine Druckversorgungseinrichtung bzw. eine Druckluftversorgungseinrichtung, die hier stationär angeordnet ist. Diese Druckluftversorgungseinrichtung kann insbesondere aber nicht ausschließlich das Druckluftreservoir 14 mit Druckluft versorgen.

Die Druckluft von der Druckluftversorgungseinrichtung 4 kann über einen Drehverteiler 7 auf die einzelnen Umformungsstationen 2 verteilt werden. Die Druckluftversorgungseinrichtung 4 kann etwa an einer Luftdruckquelle wie einem Kompressor angeschlossen sein.

Figur 2 zeigt eine Druckversorgungseinrichtung in einer ersten Ausgestaltung. Diese weist eine Be- und Entlüftungseinheit 42 auf, welcher über einen Zulauf 72 Druckluft zugeführt werden kann und welche über einen Anschluss 74 bzw. eine Ableitung 74 die Druckluft an die eigentliche Vorrichtung zum Umformen der Kunststoffvorformlinge zu Kunststoffbehältnissen abgeben kann.

Zusätzlich weist diese Druckluftversorgungseinrichtung hier zwei Filtereinrichtungen 44 und 46 auf, wobei diese Filtereinrichtungen als Reinfilter unterschiedlicher Größe ausgebildet sein können. Diese beiden Filtereinrichtungen 44 und 46 können dabei alternativ Einsatz finden.

Das Bezugszeichen 48 kennzeichnet eine Vebrauchsmesseinrichtung, welche den Druckluftverbrauch messen kann. Diese Verbrauchsmessung kann hier ebenfalls in die modulare Druckluftversorgungseinheit bzw. das modulare Luftwandkonzept integriert werden. Insbesondere wird dabei diese Verbrauchsmesseinrichtung 48 in den Primäranschluss der Druckluftversorgungseinrichtung integriert.

Es ist dabei auch möglich, dass die Vebrauchsmesseinrichtung in unterschiedlichen Größen ausgeführt werden kann. Eine erste Größe könnte beispielsweise bis 3100 Nm³/h beispielsweise bei 40 bar reichen und eine zweite Größe bis 6200 Nm³/h beispielsweise bei einem Druck von 40 bar. Daneben ist aber auch eine spätere Umrüstung auf höhere Volumenströme jederzeit möglich. Die Druckluftversorgungseinrichtung weist daher bevorzugt Anschlussmittel bzw. Anschlüsse auf, um unterschiedliche Verbrauchsmesseinrichtungen integrieren zu können.

Es wird dabei darauf hingewiesen, dass die beiden Feinfilter unterschiedlicher Größe 44 und 46 auch gegeneinander ausgetauscht werden können, um so die Maschinen an unterschiedliche Volumenströme anzupassen. Auf diese Weise wird, wie oben erwähnt, die unterschiedliche Einsetzbarkeit der Druckversorgungseinrichtung erhöht.

Bei der in Figur 3 gezeigten Ausgestaltung sind hier auch zwei unterschiedliche Aktivkohle- und Staubfilter 84 und 86 vorgesehen. Auch hier sind diese Filter in unterschiedlicher Größe vorgesehen und können an den jeweiligen Bedarf der Maschine angepasst werden.

Die Bezugszeichen 88 und 90 kennzeichnen zwei Vorfilter, die hier ebenfalls für unterschiedliche Größen ausgestaltet werden können. Damit kann auch hier die Vorrichtung schnell auf unterschiedliche Verbrauchkriterien angepasst werden. Auch diese beiden Vorfilter 88 und 90 sind alternativ einsetzbar.

Es ist dabei möglich, dass der von den Zuleitungen 72 kommende Luftstrom sämtliche dieser genannten Filter durchläuft. Man erkennt auch, dass hier insbesondere die Aktivkohle- und Staubfilter in ein bestehendes Konzept integriert werden können. Damit erkennt man weiterhin, dass die Druckluftversorgungseinrichtung durch unterschiedliche Module hier mit den Bezugszeichen M1 und M2 ergänzt werden kann.

So bezeichnet hier das Modul M1 ein Modul, welches Aktivkohlefilter und Staubfilter in unterschiedlichen Größen aufweisen kann. Das Bezugszeichen M2 kennzeichnet ein Modul, welches hier zwei Vorfilter in unterschiedlichen Größen aufweisen kann und dazu einen Kondensatabscheider 92. Es wird jedoch darauf hingewiesen, dass dieser Kondensatabscheider auch an anderen Modulen angesetzt werden kann.

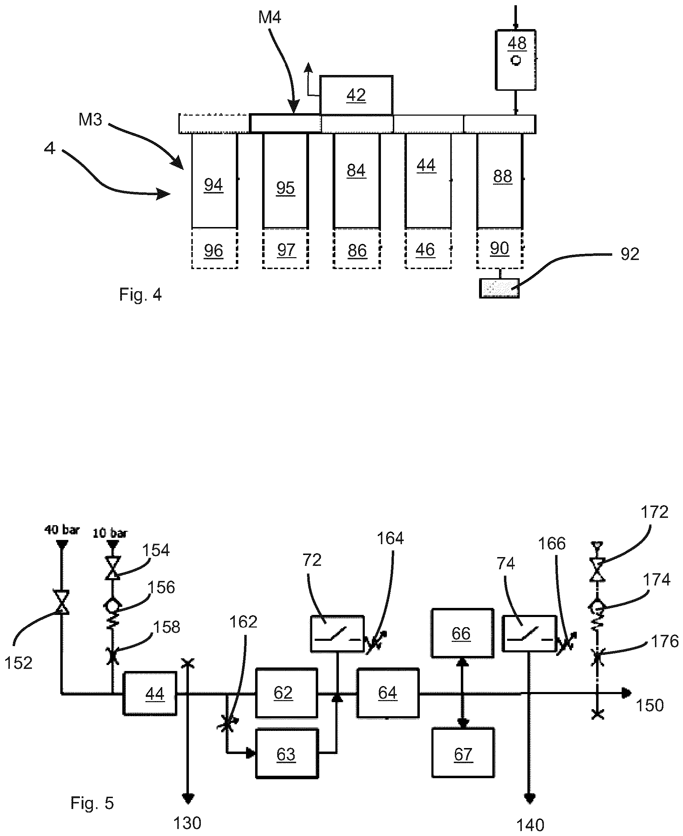

Bei der in Figur 4 gezeigten Ausführungsform ist die Druckluftversorgungseinrichtung und zwei weitere Module M3 und M4 erweitert. Das Modul M3 weist dabei hier zwei Sterilfilter 94 und optional 96 in unterschiedlicher Größe auf. Auch das Modul M4 weist zwei Sterilfilter 95 und 97 auf.

Besonders bevorzugt sind allgemein die Sterilfilter 94 - 97 stromabwärts bezüglich Be- und Entlüftungseinheit 42 angeordnet. Dies ist vorteilhaft wenn die Be- und Entlüftungseinheit nicht steril ausführbar ist.

Figur 5 zeigt ein Funktionsschema für eine Druckluftversorgungseinrichtung. Dabei sind hier zwei Druckluftzuführungseinrichtungen vorgesehen, die beispielsweise eine Druckluft von 40 bar und 10 bar zur Verfügung stellen können. Die Druckluft kann beispielsweise von Kompressoren erzeugt werden. Die Bezugszeichen 152 und 154 bezeichnen hier manuelle Absperrhähne, die für diese beiden Druckluftzuführungen dienen. Das Bezugszeichen 156 kennzeichnet ein Rückschlagventil, welches verhindert, dass Druckluft aus der 40 bar Leitung in die 10 bar Leitung gepresst werden kann.

Das Bezugszeichen 158 kennzeichnet eine Drosseleinrichtung, genauer gesagt hier eine Festblendeneinrichtung.

An diese Druckluftzuführung schließt sich eine erste Filtereinheit 44 an. An diese erste Filtereinrichtung kann sich ein Niederdruckanschluss anschließen, der beispielsweise Dauerluft zur Verfügung stellen kann, welche beispielsweise zum Schalten von pneumatischen Ventilen dienen kann.

Das Bezugszeichen 162 kennzeichnet eine Drosseleinrichtung und insbesondere eine einstellbare Drossel oder auch eine Festblende, welche bevorzugt für eine Softstartfunktion dient.

Die Bezugszeichen 62 und 63 kennzeichnen Ventile und insbesondere kennzeichnet das Bezugszeichen 62 ein 2/2 Wegeventil und das Bezugszeichen 63 ein 3/2 Wegeventil, welches für die Softstartfunktion dient. Dieses Ventil ist in seiner Grundstellung geschlossen (NC: normally closed). Das Bezugszeichen 72 kennzeichnet einen Druckschalter, der hier zum Überwachen der Ventileinrichtungen 62 und 63 dient. Dieser kann analog oder digital ausgeführt sein und kann auch Initiatoren oder allgemein Sensoreinrichtungen aufweisen, welche Stellungsabfragen der Ventile 62 und 63 ermöglichen.

Das Bezugszeichen 64 kennzeichnet eine weitere Ventileinrichtung, hier insbesondere ein 2/2 Wegeventil zum Sperren der NC.

Die Bezugszeichen 66 und 67 kennzeichnen zwei weitere Ventileinrichtungen, welche hier den Entlüftungsvorgängen dienen.

Das Bezugszeichen 74 kennzeichnet einen Druckschalter, der ebenfalls wieder analog, digital oder mit Initiatoren bzw. allgemein Sensoreinrichtungen ausgestattet sein kann, um Stellungsanfragen an die Ventile 64, 66 und 67 zu richten. Entsprechend kennzeichnet das Bezugszeichen 140 einen zweiten Niederdruckanschluss, der jedoch hier aktiv wegschaltbar ist.

Das Bezugszeichen 150 kennzeichnet den Hauptdruckanschluss, der den Hochdruck an die Vorrichtung zum Umformen von Kunststoffvorformlingen zu Kunststoffbehältnissen liefert.

Die Bezugszeichen 172, 174 und 176 können Bestandteile eines alternativen Niederdruckanschlusses sein, über den beispielsweise Dauerluft zur Verfügung gestellt werden kann. Dabei kennzeichnet das Bezugszeichen 172 wiederum einen manuellen Absperrhahn, das Bezugszeichen 174 ein Rückschlagventil und das Bezugszeichen 176 ebenfalls wieder eine Drosseleinrichtung oder eine Festblende.

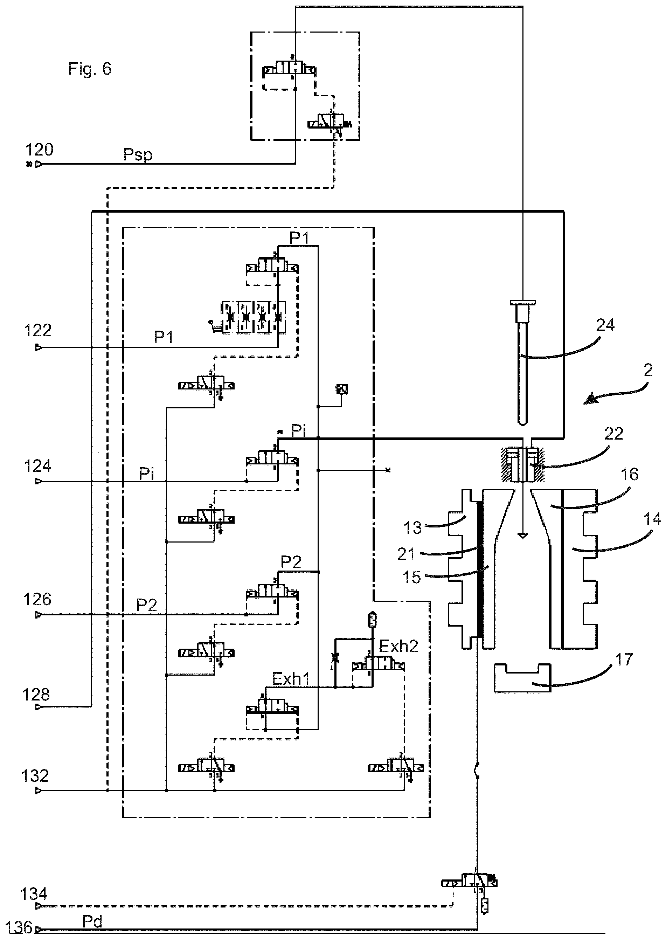

Fig. 6 zeigt eine schematische Darstellung einer erfindungsgemäßen Vorrichtung. Dabei kennzeichnet das Bezugszeichen 2 eine Umformungsstation. Diese weist zwei Seitenteilträger 13, 14 auf, an denen jeweils Seitenteile 15, 16 einer Blasform angeordnet sind. Das Bezugszeichen 17 kennzeichnet ein Bodenteil der Blasform. Zwischen dem Seitenteilträger 13 und dem Seitenteil 15 ist ein optionales Druckkissen vorgesehen, welches im Arbeitsbetrieb die beiden Seitenteile aufeinander zu drängt, um einen Formspalt zu verringern.

Das Bezugszeichen 24 kennzeichnet eine Reckstange, die hier als Spülstange ausgebildet ist und über welche den Kunststoffvorformlingen ebenfalls Luft zugeführt werden kann (insbesondere auch zum Kühlen des Behältnisbodens.

Die Bezugszeichen 122, 124, 126, 128 und 132 kennzeichnen Reservoirs für unterschiedliche Druckstufen, wobei diese Reservoirs bevorzugt als Ringkanäle ausgebildet sind. Das Bezugszeichen 134 kennzeichnet ein Reservoir, welches einen Steuerdruck für die einzelnen, nicht näher bezeichneten, Ventile zur Verfügung stellt.

Die Bezugszeichen P1, Pi und P2 kennzeichnen unterschiedliche Druckstufen und das Bezugszeichen Psp den Spüldruck.

### Bezugszeichenliste

- 2: Umformungsstationen
- 4: Druckversorgungseinrichtung
- 7: Drehverteiler
- 7a: Anschlussbereich der Module
- 9: Druckluftversorgungseinrichtungen
- 10: Kunststoffvorformlinge
- 12: drehbarer Träger
- 13, 14: Seitenteilträger
- 15,16: Seitenteile der Blasform
- 17: Bodenteil der Blasform
- 20: Kunststoffbehältnisse
- 21: Druckkissen
- 22: Beaufschlagungseinrichtung
- 24: Reckstange bzw. Spülstange
- 32: Transportstern
- 42: Be- und Entlüftungseinheit
- 44: Filtereinrichtung
- 46: Filtereinrichtung
- 48: Verbrauchsmesseinrichtung
- 50: Erwärmungseinrichtung
- 52: Erwärmungseinrichtungen
- 54: Transporteinrichtung
- 62: Ventileinrichtung
- 63: Ventileinrichtung
- 64: Ventileinrichtung
- 66: Ventileinrichtung
- 67: Ventileinrichtung
- 72: Zulauf, Zuführleitung, Druckschalter
- 74: Anschluss, Druckschalter, Abführleitung, Zuführleitung
- 84: Aktivkohlerfilter
- 86: Staubfilter
- 88: Vorfilter
- 90: Vorfilter
- 92: Kondensatabscheider
- 94: Sterilfilter
- 95: Sterilfilter
- 96: Sterilfilter
- 97: Sterilfilter
- 120: Reservoir für Spülluft
- 122: Reservoir für P1 - Druck
- 124: Reservoir für Pi Druck
- 126: Reservoir für P2 - Druck
- 128: Reservoir
- 140: Niederdruckanschluss
- 150: Hauptdruckanschluss
- 152: Absperrhahn
- 154: Absperrhahn
- 156: Rückschlagventil
- 158: Drosseleinrichtung
- 162: Drosseleinrichtung
- 172: manueller Absperrhahn
- 174: Rückschlagventil
- 176: Drosseleinrichtung, Festblende
- M1, M4: Modul
- Psp: Spüldruck
- P1, Pi P2,: Druckstufen

## Patentansprüche

1. Vorrichtung zum Umformen von Kunststoffvorformlingen (10) zu Kunststoffbehältnissen (20) mit wenigstens einer Umformungsstation (2), welche eine Blasform (8) aufweist, innerhalb derer die Kunststoffvorformlinge (10) durch Beaufschlagung mit einem fließfähigen Medium zu den Kunststoffbehältnissen (20) umformbar sind, und mit einer Beaufschlagungseinrichtung (22) welche die Kunststoffvorformlinge mit dem fließfähigen Medium beaufschlagt, wobei die Vorrichtung eine Druckluftversorgungseinrichtung (4) aufweist, welche das fließfähige Medium zur Verfügung stellt und diese Druckluftversorgungseinrichtung (4) stationär angeordnet ist, und die Druckluftversorgungseinrichtung (4) eine Leckageerkennungseinrichtung aufweist, welche dazu geeignet und bestimmt ist, Leckagen in der Druckluftversorgung der Vorrichtung (1) zu ermitteln
**dadurch gekennzeichnet, dass**
die Leckageerkennungseinrichtung (120) eine Neiderdruckluftversorgungseinrichtung aufweist und eine Überprüfung auf Leckagen mit einem Niederdruck erfolgt, der unter 15 bar liegt.

2. Vorrichtung (1) nach Anspruch 1,
**dadurch gekennzeichnet, dass**
die Niederdruckluftversorgungseinrichtung mit der Druckluftversorgung der Vorrichtung verbindbar ist.

3. Vorrichtung nach wenigstens einem der vorangegangenen Ansprüche 1 - 2,
**dadurch gekennzeichnet, dass**
die Niederdruckversorgungseinrichtung aktivierbar und deaktivierbar ist.

4. Vorrichtung (1) nach wenigstens einem der vorangegangenen Ansprüche,
**dadurch gekennzeichnet, dass**
die Leckageerkennungseinrichtung eine Druckanschlusseinrichtung aufweist, wobei diese Druckanschlusseinrichtung einen gegenüber einer Hauptdruckanschlusseinrichtung verkleinerten Querschnitt aufweist.

5. Vorrichtung (1) nach wenigstens einem der vorangegangenen Ansprüche,
**dadurch gekennzeichnet, dass**
die Vorrichtung (1) wenigstens eine erste Filtereinrichtung (44) aufweist.

6. Vorrichtung (1) nach wenigstens einem der vorangegangenen Ansprüche,
**dadurch gekennzeichnet, dass**
die Druckluftversorgungseinrichtung (4) eine Vielzahl von Luftventileinrichtungen (62, 63 64, 66, 67) aufweist und diese Luftventileinrichtungen (62, 64, 66, 68) bevorzugt aus einer Gruppe von Ventileinrichtungen ausgewählt sind, welche Kolbenschieberventile, Sitzventile, Kugelhahnventile und dergleichen enthält.

7. Vorrichtung nach dem vorangegangenen Anspruch,
**dadurch gekennzeichnet, dass**
die Vorrichtung eine Überwachungseinrichtung (72, 74) zum Überwachen der Luftventileinrichtungen (62, 63, 64, 66, 67) aufweist, wobei die Überwachungseinrichtung ein analoger oder digitaler Druckschalter ist.

8. Vorrichtung (1) nach wenigstens einem der vorangegangenen Ansprüche,
**dadurch gekennzeichnet, dass**
die Vorrichtung (1) eine Verschließeinrichtung zum Verschließen der Beaufschlagungseinrichtung aufweist, welche einen Schwenkmechanismus aufweist, der einen Verschließkörper in einen Verfahrweg der Beaufschlagungseinrichtung schwenkt.

9. Verfahren zum Betreiben einer Vorrichtung (1) zum Umformen von Kunststoffvorformlingen (10) zu Kunststoffbehältnissen (20) nach Anspruch 1, wobei Kunststoffvorformlinge (10) mit wenigstens einer Umformungsstation (2) zu den Kunststoffbehältnissen (20) umgeformt werden, wobei die Umformungsstation (2) eine Blasform aufweist, innerhalb derer die Kunststoffvorformlinge (10) durch Beaufschlagung mit dem fließfähigen Medium zu den Kunststoffbehältnissen (20) umgeformt werden, und wobei die Kunststoffvorformlinge mit einer Beaufschlagungseinrichtung (22) mit dem fließfähigen Medium beaufschlagt werden, wobei mit einer Druckluftversorgungseinrichtung (4) das fließfähige Medium zur Verfügung gestellt wird und diese Druckluftversorgungseinrichtung (4) stationär angeordnet ist,
**dadurch gekennzeichnet, dass**
wenigstens zeitweise die Vorrichtung (1) mit der Druckluftversorgungseinrichtung auf Leckagen hin überprüft wird.

10. Verfahren nach dem vorangegangenen Anspruch,
**dadurch gekennzeichnet, dass**
in aufeinanderfolgenden Verfahrensschritten unterschiedliche Bestandteile der Vorrichtung auf Leckagen hin überprüft werden.

11. Verfahren nach dem vorangegangenen Anspruch, **gekennzeichnet durch** die nachfolgenden Verfahrensschritte:
- Überprüfen einer Luftdrehverteilereinrichtung
- Überprüfen von Regelventilen und insbesondere Domdruckregelventilen;
- Überprüfen von Druckluftspeichereinrichtungen, insbesondere Ringkanälen;
- Überprüfen von Dichtungseinrichtungen.

## Claims

1. Apparatus for forming plastic preforms (10) into plastic containers (20), having at least one forming station (2) which has a blow mold (8) within which the plastic preforms (10) can be formed into the plastic containers (20) by being acted upon by a flowable medium, and having an application device (22) which applies the plastic preforms with the flowable medium, wherein the apparatus has a compressed air supply device (4) which provides the flowable medium and this compressed air supply device (4) is arranged stationary, and the compressed air supply device (4) has a leakage detection device which is suitable and intended for detecting leaks in the compressed air supply of the apparatus (1), **characterized in that**
the leakage detection device (120) has a low pressure air supply device and a check for leaks is carried out at a low pressure of less than 15 bar.

2. Apparatus (1) according to claim 1,
**characterized in that**
the low-pressure air supply device can be connected to the compressed air supply of the apparatus.

3. Apparatus according to at least one of the preceding claims 1 - 2, **characterized in that**
the low-pressure supply device can be activated and deactivated.

4. Apparatus (1) according to at least one of the preceding claims, **characterized in that**
the leakage detection device has a pressure connection device, wherein this pressure connection device has a reduced cross-section compared to a main pressure connection device.

5. Apparatus (1) according to at least one of the preceding claims, **characterized in that**
the apparatus (1) has at least one first filter device (44).

6. Apparatus (1) according to at least one of the preceding claims, **characterized in that**
the compressed air supply device (4) comprises a plurality of air valve devices (62, 63, 64, 66, 67) and these air valve devices (62, 64, 66, 68) are preferably selected from a group of valve devices which includes piston spool valves, poppet valves, ball valve valves and the like.

7. Apparatus according to the preceding claim,
**characterized in that**
the apparatus has a monitoring device (72, 74) for monitoring the air valve devices (62, 63, 64, 66, 67), wherein the monitoring device is an analogue or digital pressure switch.

8. Apparatus (1) according to at least one of the preceding claims, **characterized in that**
the apparatus (1) has a closing device for closing the application device, which has a pivoting mechanism which pivots a closing body into a travel path of the application device.

9. Method for operating an apparatus (1) for forming plastic preforms (10) into plastic containers (20) according to claim 1, wherein plastic preforms (10) are formed into the plastic containers (20) by at least one forming station (2), wherein the forming station (2) comprises a blow mold, within which the plastic preforms (10) are formed into the plastic containers (20) by application with the flowable medium, and wherein the plastic preforms are applied with the flowable medium by an application device (22), wherein the flowable medium is made available by a compressed air supply device (4) and this compressed air supply device (4) is arranged in a stationary manner,
**characterized in that**
the apparatus (1) is at least temporarily checked for leaks with the compressed air supply device.

10. Method according to the preceding claim,
**characterized in that**
different components of the apparatus are checked for leaks in successive process steps.

11. Method according to the preceding claim, **characterized by** the following method steps:
- checking an air rotary distributor device
- checking control valves and in particular dome pressure control valves;
- checking compressed air storage devices, in particular ring channels;
- checking sealing devices.

## Revendications

1. Dispositif de façonnage de préformes en matière plastique (10) en des récipients en matière plastique (20) avec au moins une station de façonnage (2), laquelle présente un moule de soufflage (8), à l'intérieur duquel les préformes en matière plastique (10) peuvent être façonnées en les récipients en matière plastique (20) par exposition à l'action d'un milieu pouvant s'écouler, et avec un système de sollicitation (22), lequel expose les préformes en matière plastique à l'action du milieu pouvant s'écouler, dans lequel le dispositif présente un système d'alimentation en air comprimé (4), lequel met à disposition le milieu pouvant s'écouler et lequel système d'alimentation en air comprimé (4) est disposé de manière stationnaire, et le système d'alimentation en air comprimé (4) présente un système de détection de fuites, lequel est adapté pour et se destine à déterminer des fuites dans l'alimentation en air comprimé du dispositif (1),
**caractérisé en ce que**
le système de détection de fuites (120) présente un dispositif d'alimentation en air à basse pression et une vérification de présence éventuelle de fuites est effectuée à une basse pression, qui est inférieure à 15 bar.

2. Dispositif (1) selon la revendication 1,
**caractérisé en ce que**
le système d'alimentation en air à basse pression peut être relié à l'alimentation en air comprimé du dispositif.

3. Dispositif selon au moins l'une quelconque des revendications précédentes 1 - 2,
**caractérisé en ce que**
le système d'alimentation en air à basse pression peut être activé et désactivé.

4. Dispositif (1) selon au moins l'une quelconque des revendications précédentes,
**caractérisé en ce que**
le dispositif de détection de fuites présente un système de raccordement sous pression, dans lequel ledit système de raccordement sous pression présente une section transversale réduite par rapport à un système de raccordement sous pression principal.

5. Dispositif (1) selon au moins l'une quelconque des revendications précédentes,
**caractérisé en ce que**
le dispositif (1) présente au moins un premier système de filtre (44).

6. Dispositif (1) selon au moins l'une quelconque des revendications précédentes,
**caractérisé en ce que**
le système d'alimentation en air comprimé (4) présente une pluralité de systèmes à soupape d'air (62, 63, 64, 66, 67) et ces systèmes à soupape d'air (62, 64, 66, 68) sont choisis de manière préférée parmi un groupe de systèmes de soupape, lequel contient des soupapes à pistons, des soupapes à siège, des soupapes à boisseau sphérique et similaires.

7. Dispositif selon la revendication précédente,
**caractérisé en ce que**
le dispositif présente un système de surveillance (72, 74) destiné à surveiller les systèmes à soupape d'air (62, 63, 64, 66, 67), dans lequel le système de surveillance est un commutateur manométrique analogique ou numérique.

8. Dispositif (1) selon au moins l'une quelconque des revendications précédentes,
**caractérisé en ce que**
le dispositif (1) présente un système de fermeture destiné à fermer le système de sollicitation, lequel présente un mécanisme de pivotement, qui fait pivoter un corps de fermeture sur un trajet de déplacement du système de sollicitation.

9. Procédé pour faire fonctionner un dispositif (1) de façonnage de préformes en matière plastique (10) en récipients en matière plastique (20) selon la revendication 1, dans lequel des préformes en matière plastique (10) sont façonnées en les récipients en matière plastique (20) avec au moins une station de façonnage (2), dans lequel la station de façonnage (2) présente un moule de soufflage, à l'intérieur duquel les préformes en matière plastique (10) sont façonnées en les récipients en matière plastique (20) par exposition à l'action du milieu pouvant s'écouler, et dans lequel les préformes en matière plastique sont exposées à l'action du milieu pouvant s'écouler avec un système de sollicitation (22), dans lequel le milieu pouvant s'écouler est mis à disposition avec un système d'alimentation en air comprimé (4) et ce système d'alimentation en air comprimé (4) est disposé de manière stationnaire,
**caractérisé en ce que**
la présence éventuelle de fuites sur le dispositif (1) est vérifiée au moins par intermittence avec le système d'alimentation en air comprimé.

10. Procédé selon la revendication précédente,
**caractérisé en ce que**
la présence éventuelle de fuites sur différents éléments constitutifs du dispositif est surveillée dans des étapes de procédé se suivant les unes les autres.

11. Procédé selon la revendication précédente, **caractérisé par** les étapes de procédé qui suivent :
- la surveillance d'un système de répartition par rotation d'air ;
- la surveillance de soupapes de régulation et en particulier de soupapes de régulation de pression de pression à dôme ;
- la surveillance de systèmes d'accumulation d'air comprimé en particulier de canaux annulaires ;
- la surveillance de systèmes d'étanchéité.
